# FASCICULE DE BREVET EUROPEEN

(11) **EP 0 850 082 B1**
(45) Date de publication et mention de la délivrance du brevet: **08.03.2000**
(21) Numéro de dépôt: 96931003.6
(22) Date de dépôt: 03.09.1996
(51) Int. Cl.: A61M 15/00, A61J 1/03, B65D 75/36

(54) **DISPOSITIF DE PRE-DOSAGE DE PRODUIT PULVERULENT POUR UN DISTRIBUTEUR DE PRODUIT**
VORRICHTUNG ZUR VOR-DOSIERUNG EINES PULVERFÖRMIGEN PRODUKTS FÜR EINE PRODUKTABGABEVORRICHTUNG
DEVICE FOR THE PRE-DOSING OF A POWDERY PRODUCT FOR A PRODUCT DISPENSER

(30) Priorité: 04.09.1995 FR 9510344
(43) Date de publication de la demande: 01.07.1998
(73) Titulaire: TEBRO S.A., L-1118 Luxembourg (LU)
(72) Inventeur: STRADELLA, Guiseppe, I-1118 Camogli (IT)
(74) Mandataire: CAPRI SARL
(86) Numéro de dépôt international: EP9603849
(87) Numéro de publication internationale: WO9709082

(56) Documents cités:
- EP-A- 0 469 814
- WO-A-93/16748
- DE-A- 4 400 083
- DE-A- 4 400 084
- GB-A- 2 270 293

## Description

L'invention concerne un dispositif de pré-dosage de produit pulvérulent pour un distributeur de produit, et plus particulièrement pour un inhalateur.

Par inhalateur, on entend tous les dispositifs d'inhalation, qu'ils soient actionnés manuellement ou par l'inhalation de l'utilisateur ; qu'ils soient passifs, c'est à dire où le produit est expulsé par un écoulement d'air créé par l'utilisateur, ou actifs, c'est à dire où l'écoulement d'air est créé par l'appareil. L'invention s'applique particulièrement aux inhalateurs de poudre sèche.

Dans le domaine des inhalateurs de poudre sèche, il existe principalement deux familles de dispositifs de distribution de produits pulvérulents couramment utilisées :
- les dispositifs de type à réservoir multi-doses, où la poudre est contenue dans un seul réservoir commun, l'appareil étant pourvu de moyens de dosage appropriés pour distribuer, à chaque actionnement une quantité déterminée de poudre.
- les dispositifs de type à pré-dosage, où chaque dose de poudre est contenue dans des cavités séparées, fermées hermétiquement.

Les dispositifs de type à pré-dosage sont aujourd'hui particulièrement intéressants pour les poudres ayant des molécules fragiles, par exemple formés de longues chaînes qui sont facilement cassables pendant la manipulation, et nécessitent une grande stabilité. Ceci ne peut être obtenu qu'avec une véritable séparation de la poudre de l'atmosphère, ce qui est typique des emballages à dose unique pré-dosés séparément.

Pour cette application, les dispositifs d'emballage communément appelé "blister" se sont avérés être particulièrement adaptés. En effet, de par sa configuration, qui utilise un support pourvu de plusieurs cavités de doses séparées et fermées hermétiquement, par exemple par une feuille d'aluminium, le blister permet d'obtenir une séparation hermétique parfaite de chaque dose.

Toutefois, l'utilisation de blister est aujourd'hui limitée par de nombreux problèmes qui sont apparus lors de son utilisation.

Un des inconvénients principaux est dû au fait que, pour rendre la dose disponible à l'inhalation, il est nécessaire d'utiliser un inhalateur muni d'un dispositif capable d'ouvrir l'emballage (peler, casser ou déchirer l'aluminium) d'une manière efficace.

Actuellement les solutions suivantes sont utilisées pour obtenir ce résultat :
- L'inhalateur comprend des moyens de pelage capable de peler la couche fermant les cavités de doses. Un tel dispositif est par exemple décrit dans le document FR-2 660 550. Ce type de dispositif de pelage est toutefois très compliqué à fabriquer et à assembler et est donc très coûteux.
- L'inhalateur est muni d'une aiguille utilisée pour percer l'aluminium juste avant la distribution de la dose. Cette solution présente le désavantage de ne fournir qu'une ouverture partielle de la cavité contenant la poudre, et en outre de replier vers l'intérieur l'aluminium dans la cavité de dose. Il en résulte que des parties de dose restent couvertes par la feuille d'aluminium et ne peuvent par conséquent pas être expulsées totalement par le courant d'air produit par l'utilisateur ou par l'inhalateur lors de l'inhalation. Il s'ensuit une grande difficulté à garantir une reproductibilité de dose efficace car souvent la totalité de la dose n'est pas distribuée.
- L'inhalateur est pourvu d'un moyen, quelque peu similaire à un petit doigt, qui est utilisé pour presser contre le côté plastique de la cavité du blister pour créer dans la cavité une pression suffisante pour casser la feuille d'aluminium et ainsi déplacer la poudre, dans le canal d'expulsion. Cette solution présente également un inconvénient. En effet, la pression exercée sur la poudre, combinée avec la résistance offerte par la feuille d'aluminium avant qu'elle ne casse, crée une compression importante de la poudre, ce qui à tendance à agglomérer ladite poudre en gros morceaux. Le document EP-0 469 814 divulgue un tel dispositif.
- Dans le document DE-44 00 083, il est divulgué un dispositif de blister dont les cavités sont pourvues de moyens de percement permettant de réduire la compression de la poudre. Ces moyens de percement agissent de l'intérieur de la cavité et percent la membrane vers l'extérieur. Toutefois, ce dispositif ne fournit également qu'une ouverture partielle ou irrégulière de la membrane d'aluminium et prévoit même dans certains cas de détacher complètement ladite membrane de la cavité. Ceci peut ne pas être gênant dans certaines applications des blisters, mais ce n'est pas compatible et même dangereux dans les dispositifs d'inhalation où une dose de poudre est d'abord transférée d'un blister dans une chambre d'expulsion avant d'être inhalée. En effet, outre le désavantage mentionné précédemment de non reproductibilité parfaite de la dose, des parties de la membrane déchirée irrégulièrement ou arrachées risquent de réduire ou de bloquer l'écoulement d'air nécessaire à l'expulsion de la poudre, ou même d'être éjectées et donc inhalées avec la poudre, avec des conséquence néfastes évidentes.

Le document WO 93/16748 divulgue un dispositif de dosage permettant de remplir des réservoirs individuels contenant chacun une dose à partir d'un réservoir commun et de transférer ces réservoirs individuels dans le canal d'expulsion.

Le document GB-2 270 293 divulgue un dispositif comportant des réservoirs individuels formés de deux parties coulissant l'une dans l'autre. Ces réservoirs, de par leur construction en deux parties mobiles, ne sont pas hermétiquement fermés.

Le document DE-A-4 400 084 (voir en particulier la figure 7b), qui est considéré comme l'état de la technique le plus proche, décrit un dispositif selon le préambule de la revendication 1.

La présente invention a pour but de fournir un dispositif de pré-dosage de produits pulvérulents pour un inhalateur, qui ne présente pas les inconvénients précités.

La présente invention a aussi pour but de fournir un tel dispositif où la totalité de la dose enfermée dans l'emballage individuel est distribuée dans le canal d'expulsion de l'inhalateur .

La présente invention a encore pour but de fournir un tel dispositif dans lequel il ne se créer pas de compression et/ou de compactage de la poudre pendant le passage du réservoir individuel vers le canal d'expulsion.

La présente invention a également pour but de fournir un dispositif dans lequel le réservoir individuel est ouvert d'une manière prédéterminée sans qu'une quelconque partie dudit réservoir ne puisse se détacher, pour éviter tout obstacle à l'expulsion du produit et toute possibilité d'inhalation de parties dudit réservoir.

La présente invention à donc pour objet un dispositif de pré-dosage de produit pulvérulent comme celà est défini dans la revendication 1. Différents modes de réalisation sont définis dans les revendications dépendantes.

Selon un premier mode de réalisation de l'invention, ledit élément de transfert est réalisé sous la forme d'au moins un insert rigide disposé à l'intérieur de chaque réservoir.

Selon une variante de réalisation avantageuse de l'invention, ledit insert est creux et a une structure ouverte, la forme et les dimensions extérieures de l'insert étant telles que l'insert est en contact avec le milieu de la paroi de fermeture, sur toute la longueur de celle-ci, et que la section maximale de l'insert correspond environ à la surface de ladite paroi de fermeture. Avantageusement, l'insert peut donc être mis en place dans le réservoir après le remplissage de celui-ci avec la dose de poudre.

Avantageusement, ledit insert comporte des anneaux parallèles reliés par des segments longitudinaux.

Selon une autre variante de réalisation avantageuse de l'invention, l'insert est un ressort hélicoïdal.

Selon encore une autre variante de réalisation avantageuse de l'invention, l'insert est formé d'un tube rigide et creux qui est adapté à contenir la dose de poudre destinée à chacun des réservoirs.

Les réservoirs peuvent être disposés de toutes les manières appropriées sur toutes formes de support, par exemple radialement sur un disque rigide ou souple, ou parallèlement les uns aux autres sur une bande, de préférence souple.

D'autres caractéristiques et avantages de l'invention apparaîtront au cours de la description détaillée suivante donnée à titre d'exemple non limitatif en regard des dessins joints, sur lesquels :
- les figures 1a et 1b sont des vues schématiques respectivement en perspective et en coupe verticale, d'un dispositif de pré-dosage selon l'invention,
- la figure 1c est une vue schématique en perspective d'une autre forme de réalisation d'un dispositif de pré-dosage selon l'invention,
- les figures 2 à 7 représentent des vues schématiques en perspective de différentes variantes de l'insert selon l'invention,
- la figure 8 représente une mise en oeuvre du dispositif de pré-dosage selon l'invention dans un exemple particulier d'inhalateur,
- la figure 9 est une vue similaire à celle de la figure 9 après actionnement du dispositif de transfert; et
- les figures 10 et 11 sont des vues schématiques en coupe verticale d'un blister selon un mode de réalisation de l'invention, respectivement avant et après transfert du produit dans la chambre d'expulsion.

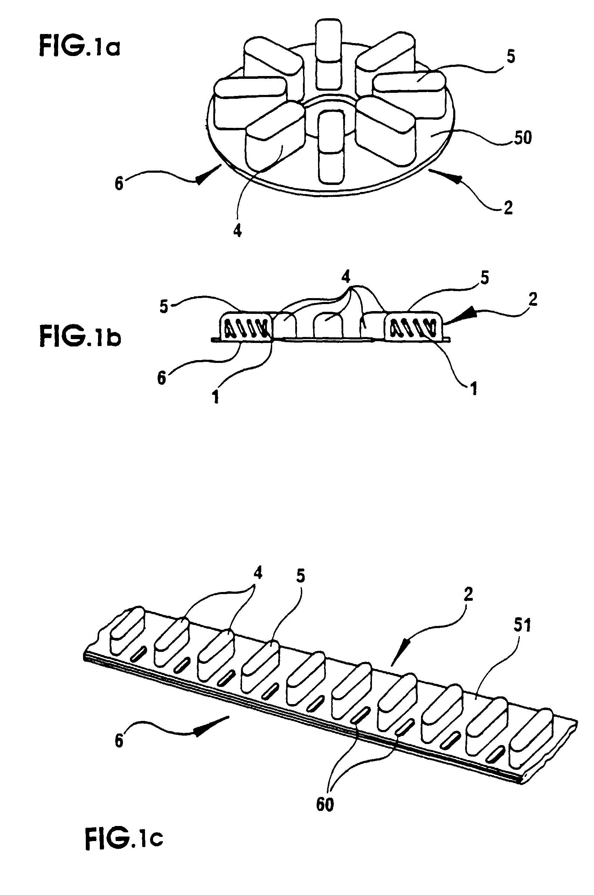

En référence aux figures 1a et 1b, il est représenté un exemple d'un emballage 2 communément appelé "blister", comportant huit réservoirs individuels 4 formés avantageusement d'une coquille , par exemple en plastique ou en aluminium, et renfermant chacun une dose de produit. Les huit réservoirs 4 sont fermés hermétiquement par une paroi de fermeture 6, de préférence une feuille d'aluminium disposée sous l'emballage 2. Bien entendu, le blister peut avoir une forme quelconque différente de celle représentée sur les figures 1a et 1b, où il est sensiblement circulaire et où les réservoirs sont disposés sensiblement radialement sur un disque rigide 50 dudit blister 2. Ainsi le nombre de réservoirs 4 peut être différent et les réservoirs, au lieu de comporter une coquille 5 recouverte d'un seul côté par la feuille d'aluminium 6 peuvent aussi comporter des cavités ouvertes des deux côtés fermées hermétiquement par deux feuilles d'aluminium inférieure et supérieure. D'autre part, les réservoirs 4 du blister 2 peuvent aussi être disposés parallèlement les uns aux autres sur une bande 51, de préférence une bande souple, comme représenté sur la figure 1c.

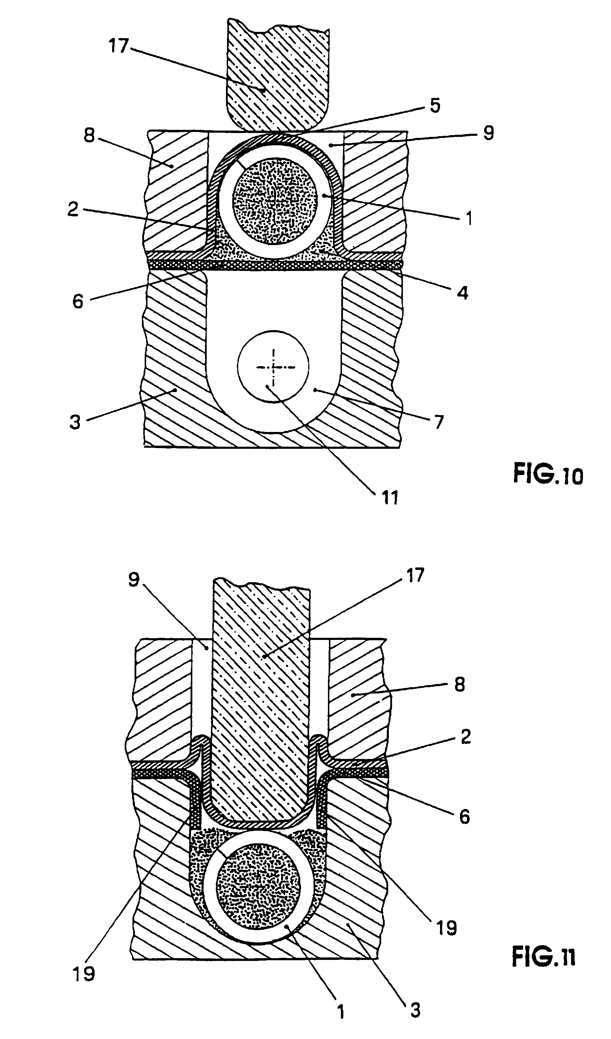

Selon un mode de réalisation avantageux de l'invention, on prévoit de disposer au moins un élément de transfert tel qu'un insert à l'intérieur de chaque réservoir 4, ledit élément de transfert t étant sensiblement rigide et ayant une forme telle que, lors d'une déformation dudit réservoir 4, il soit en contact simultanément avec deux parois opposées du réservoir, en l'occurrence dans cet exemple, avec la paroi supérieure de la cavité plastique 5 du blister 2 et avec la feuille en aluminium 6 qui ferme ladite cavité. De cette manière, lorsqu'une pression est exercée sur la coquille en plastique du blister dans le but de casser ou déchirer la feuille d'aluminium, cette pression sera directement transmise à travers cet élément de transfert rigide vers la feuille d'aluminium qui sera ainsi transpercée par l'élément de transfert, sans qu'aucune pression ne soit exercée sur la poudre. De préférence, l'élément de transfert est creux et a des dimensions extérieures correspondant sensiblement aux dimensions intérieures du réservoir 4, de telle sorte que lors d'une déformation dudit réservoir 4, ledit élément de transfert est simultanément en contact avec les côtés opposés dudit réservoir. En outre, l'élément de transfert a une telle forme qu'il est en contact avec le centre de la paroi de fermeture 6, sur toute sa longueur, et que sa section maximale correspond environ à la surface de ladite paroi de fermeture 6. Ceci apparaît notamment sur les figures 10 et 11. On obtient ainsi les résultats avantageux suivants :
- la pression initiale est transférée sur une partie prédéterminée et sur toute la longueur de la paroi de fermeture 6, ce qui entraîne une ouverture (déchirage) contrôlée et prédéterminée de celle-ci, sans qu'il n'y ait, après ouverture, ni de grandes parties de paroi qui pourrait gêner l'expulsion de la dose, ni de parties de paroi détachées complètement du réservoir et qui risquerait d'être inhalées avec la poudre.
- l'ouverture de la paroi de fermeture est progressive et totale au moment où la partie de section maximale de l'élément de transfert passe à travers l'ouverture du réservoir 4, et la totalité de la dose de poudre est donc transférée dans le canal d'expulsion de l'inhalateur.

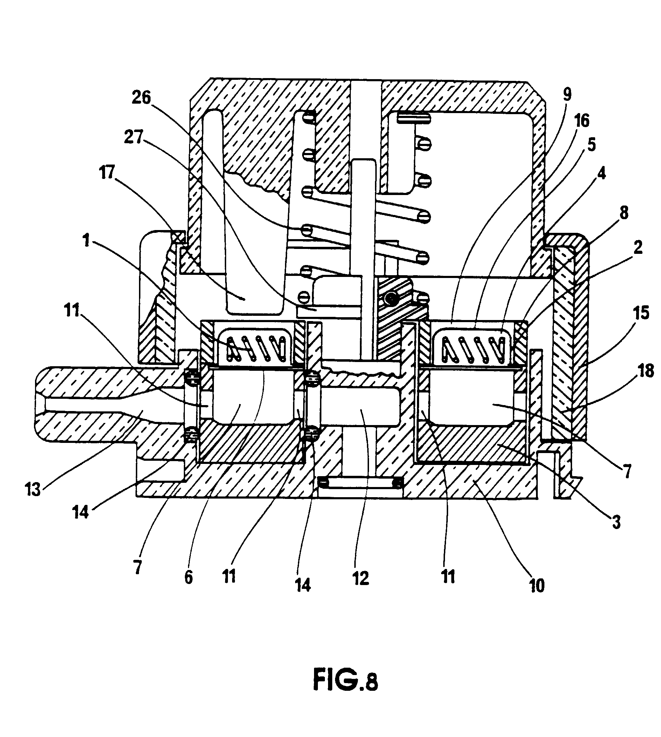

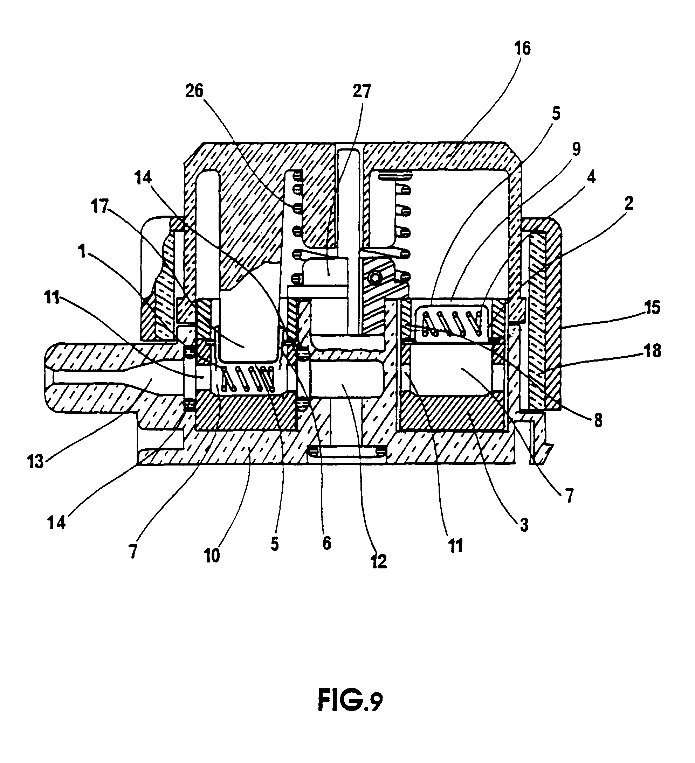

Comme visible sur les figure 1b et 2 à 7, l'élément de transfert peut être réalisé de différentes manières selon le type de la poudre, la dimension de la dose, ou la manière de remplissage du réservoir de blister. Ainsi, sur la figure 1b, l'élément de transfert 1, ci-après désigné par le terme insert, est formé simplement par un élément hélicoïdal, par exemple un ressort, qui est disposé à l'intérieur du réservoir 4. C'est ce mode de réalisation de l'insert qui est également représenté sur les figures 8 et 9 à l'aide desquelles sera décrit ci-dessous un exemple de mise en oeuvre du dispositif de pré-dosage de l'invention dans un inhalateur particulier.

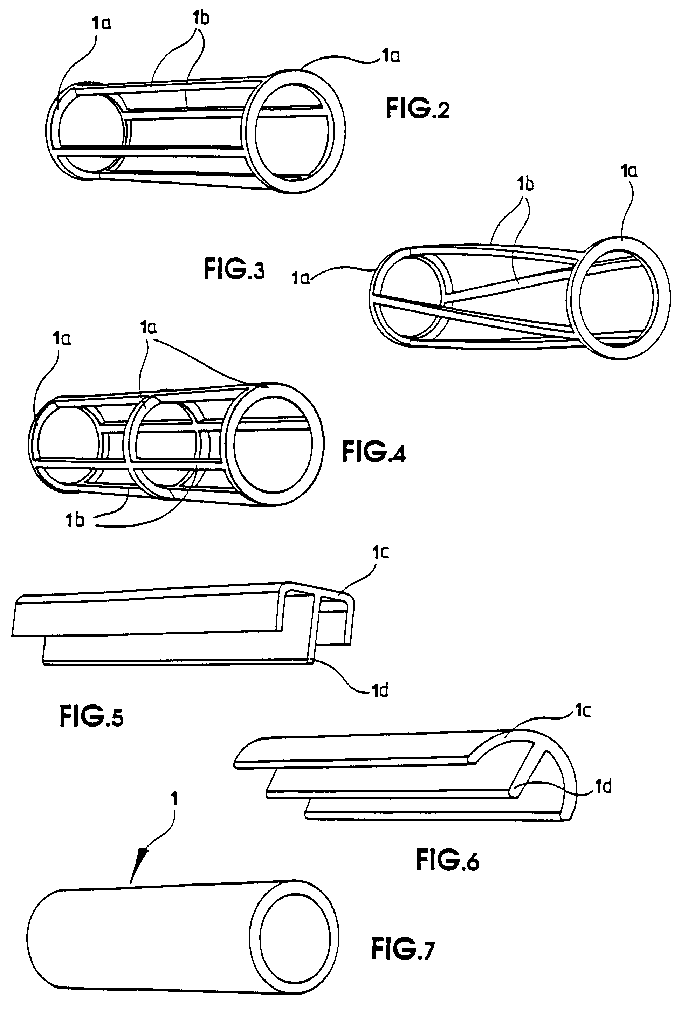

Sur les figures 2 à 4 sont représentées trois inserts différents, de forme sensiblement cylindrique, comportant une structure ouverte creuse où des anneaux parallèles la sont reliés entre eux par des segments longitudinaux 1b. Ainsi l'insert de la figure 2 comporte des segments longitudinaux 1b parallèles, alors que l'insert représenté sur la figure 3 comporte des segments longitudinaux 1b qui sont tournés axialement ou vrillés pour former une structure hélicoïdale. L'insert de la figure 4 est similaire à celui de la figure 2 mais comporte un anneau central la de renforcement et est utilisé dans le cas de plus grandes doses, où un insert plus grand est nécessaire.

Tous ces inserts mentionnés précédemment (figures 1b, 2, 3 et 4) peuvent, de par leurs structures ouvertes à segments, être appropriés pour être placés dans les réservoirs 4 de blister après le remplissage de la poudre. En effet leur structure permet de facilement les plonger à l'intérieur de la poudre après que celle-ci a été mise en place dans lesdits réservoirs du blister.

L'insert représenté sur la figure 5 comporte lui une base pleine lc ayant une section sensiblement carrée, et est destiné à être placé dans la cavité de blister avant le remplissage de la poudre. Cet insert est pourvu d'une nervure longitudinale centrale 1d appropriée pour déchirer de la même manière que les inserts cylindriques ci-dessus, la feuille d'aluminium 6 en son centre sur toute sa longueur, évitant ainsi des bords d'aluminium trop grand après leur rupture.

La figure 6 représente un insert similaire à celui de la figure 5 mais avec une base lc de section semi-cylindrique.

L'insert représenté sur la figure 7 est simplement réalisé sous la forme d'un tube plein destiné à être rempli de poudre avant son introduction dans la cavité de blister.

Ainsi les inserts représentés sur les figures 5 à 7 sont destinés à être mis en place dans la cavité de blister avant le remplissage de la poudre alors que les inserts représentés sur les figures 1b, 2 à 4 sont destinés à être mis en place après le remplissage de ladite poudre.

Bien entendu toutes les configurations mentionnées ci-dessus sont des exemples qui peuvent aisément être combinés entre eux pour former des inserts d'une forme quelconque différente, ayant par exemple des sections polygonales plutôt que cylindriques ou carrées. La caractéristique principale des inserts de l'invention est que ledit insert a une forme et des dimensions extérieures telles qu'il transmet toute pression exercée d'un côté du réservoir directement à l'autre côté, permettant ainsi le déchirement ou la rupture de la paroi de fermeture 6 (avantageusement une feuille d'aluminium) sur la totalité de sa longueur et le transfert de la totalité de la poudre du réservoir vers le canal d'expulsion d'inhalateur, sans comprimer ladite poudre.

La mise en oeuvre d'un dispositif de pré-dosage selon l'invention dans un inhalateur particulier est représentée plus complètement sur les figures 8 et 9. Bien entendu, le dispositif de pré-dosage de l'invention est adaptable à tous les inhalateurs comportant de tels dispositifs, et n'est pas limité à l'exemple représenté sur les figures 8 et 9 qui vont être décrit ci-après pour en illustrer le fonctionnement.

Dans l'exemple de mise en oeuvre de l'invention représenté sur les figures 8 et 9, le blister 2 est pourvu de cavités 4 comportant chacune une surface supérieure 5, et une ouverture opposée à ladite surface supérieure 5 et fermée hermétiquement par une feuille d'aluminium 6, le blister 2 est disposé entre un corps cylindrique 3 et un couvercle cylindrique 8. Le couvercle 8 est pourvu d'ouvertures 9 adaptées à recevoir les cavités de blister et le corps 3 et pourvu d'une manière correspondante de chambres 7 adaptées à recevoir les inserts 1 et la poudre après l'ouverture desdites cavités de blister.

Les chambres 7 sont pourvues de trous 11 qui permettent le passage d'un écoulement d'air au moment de la distribution de la poudre vers l'utilisateur de l'inhalateur.

Le corps 3, le blister 2 et le couvercle 8 sont avantageusement assemblés de manière rigide pour former une seule unité. Cette unité est placée à l'intérieur d'un évidement cylindrique approprié formé dans un corps principal 10 de l'inhalateur d'une telle manière qu'elle peut être tournée par rapport audit corps principal 10. Le corps principal 10 est pourvu d'un passage pour l'écoulement d'air, ou canal d'expulsion 12, qui traverse la chambre 7 via les trous 11 lorsque le corps 3 est placé dans une position de distribution de la poudre. Le canal d'expulsion 12 s'étend ensuite en direction d'une embouchure 13 par laquelle le produit sera délivré à l'utilisateur. Lorsque la chambre 7 est placée dans sa position de distribution, elle est munie de chaque côté d'un joint d'étanchéité 14 respectif disposé autour d'un trou 11 respectif, pour empêcher un dispersion de l'écoulement d'air lors de l'inhalation et ainsi une diminution de l'efficacité de l'inhalateur.

Le corps principal 10 porte un couvercle externe 15 fixé audit corps 10 par des moyens appropriés ledit couvercle comportant un poussoir rotatif 16 déplaçable axialement. Ce poussoir 16 incorpore un dispositif de transfert de poudre permettant de transférer une dose de poudre d'une cavité 4 de blister vers le canal d'expulsion 12 de l'inhalateur. Ce dispositif de transfert comporte un doigt 17 dont les dimensions sont telles qu'il peut pénétrer dans les ouvertures 9 du couvercle 8.

La figure 8 représente l'inhalateur avec l'unité formée du corps 3, du blister 2 et du couvercle 8, positionnée de telle manière qu'une chambre 7 est placée dans la position de distribution, ce qui signifie que ses trous 11 sont mis en correspondance avec le passage d'écoulement d'air (ou canal d'expulsion de l'inhalateur) 12, et donc avec l'embouchure 13, par l'intermédiaire des joints d'étanchéité 14. La figure 8 représente également le poussoir 16 dans une position angulaire telle que le doigt 17 est placé dans sa position d'attente ce qui signifie qu'il est en correspondance avec l'ouverture 9 du couvercle 8 et donc en face de la surface supérieure 5 de la cavité 4 d'une cavité du blister 2 mentionné ci-dessus.

Pour réaliser l'ouverture du blister et donc transférer la poudre dans la chambre 7, il suffit de presser sur le poussoir 16 ; cette action déplace le poussoir vers le bas et le doigt 17 pénètre dans l'ouverture 9 du couvercle 8 et appui vers le bas sur la surface supérieure 5 de la cavité de blister 4 sans la casser. Ce mouvement est directement transféré par l'insert 1 à la feuille d'aluminium 6 qui ferme la cavité 4. Lorsque la poussée est suffisante la feuille d'aluminium 6 casse ou se déchire sur toute la longueur de l'insert. Avantageusement, la feuille d'aluminium, une fois déchirée, ne s'étend pas à l'intérieur de la chambre 7 pour éviter de boucher partiellement les trous 11 et altérer l'expulsion du produit. A cet effet, le blister 2 est disposé suffisamment au dessus de ladite chambre 7 et la feuille d'aluminium 6 est, comme expliqué précédemment, déchirée en son centre sur toute sa longueur. La continuation du mouvement axial du doigt 17 emmène l'élément de transfert 1 à travers l'ouverture de la cavité de blister 4 et détermine l'ouverture progressive et totale de la paroi de fermeture 6, au moment où la partie de section maximale de l'élément de transfert passe à travers ladite ouverture. De cette manière sont créées des parties de bord 19 de la paroi 6 déchirée qui sont de longueur contrôlée et régulière permettant un transfert de la totalité de la poudre dans la chambre 7 et une expulsion sans obstacle de cette poudre hors de ladite chambre 7, comme visible sur les figures 9 et 11. La chambre 7, lorsqu'elle contient l'insert 1 et la dose de poudre, peut être vidée d'une manière quelconque connue par un écoulement d'air crée par exemple soit par l'inhalation de l'utilisateur, soit par un moyen externe. Cet écoulement d'air s'écoule à travers la chambre 7 dans le passage d'air 12 vers l'embouchure 13 pour distribuer la dose de poudre à l'utilisateur. Pendant cette opération, l'insert 1 reste dans la chambre 7 car le diamètre des trous 11 est tel qu'il ne permet pas son expulsion.

Après l'expulsion de la dose vers l'utilisateur, le dispositif de transfert est ramené vers sa position d'attente (figure 8) et l'unité portant le blister peut être tournée d'un angle approprié pour amener une nouvelle cavité 4, chambre 7, ouverture 9 dans une position de distribution. Pour ce faire le poussoir 16 est avantageusement pourvu de moyens permettant, lors de sa remonté sous l'effet d'un ressort de rappel 26, de faire tourner ladite unité comportant le corps 3, le blister 2 et le couvercle 8 de l'angle approprié. L'appareil est alors prêt pour une autre utilisation ultérieure.

Une fois que toutes les doses ont été délivrées de la manière décrite ci-dessus en référence aux figures 8 et 9, l'unité comportant le corps 3, le blister 2 et le couvercle 8 peut être remplacée par une nouvelle unité.

Bien entendu l'inhalateur décrit ci-dessus est juste un exemple d'application du dispositif de pré-dosage de l'invention, ce concept pouvant être utilisé de plusieurs manières différentes, par exemple dans un dispositif d'inhalation où seul le blister 2 est remplacé et non pas toute l'unité comportant le corps 3, le blister 2 et le couvercle 8. Dans ce cas, l'inhalateur peut comprendre un réceptacle unique destiné à collecter les inserts après leur transfert hors du réservoir, et ce réceptacle peut être vidé lors du remplacement du blister. Cette mise en oeuvre est notamment adaptée aux blisters comportant un grand nombre de réservoirs. D'autre part, la dose de produits une fois transférée du réservoir individuel du blister vers le canal d'expulsion de l'inhalateur peut être expulsée vers l'utilisateur d'une manière quelconque.

D'autre part, le dispositif de transfert peut avoir une forme différente, notamment lorsque le blister est réalisé sous la forme d'une bande 51 (figure 1c). Ainsi, le doigt peut être remplacé par exemple par une roue qui écraserait au fur et à mesure les réservoirs disposés sur la bande. Éventuellement, la bande 51 peut comporter des trous 60 permettant son entraînement par une roue dentée ou tout autre dispositif approprié.

## Revendications

1. Dispositif de pré-dosage (2) de produit pulvérulent disposé dans un distributeur de produit comprenant un canal d'expulsion (12), le dispositif comprenant au moins un réservoir (4) renfermant chacun une dose de produit destinée à être expulsée à chaque actionnement du distributeur, ledit distributeur comprenant un dispositif de transfert (16, 17) destiné à transférer la totalité d'une dose de produit d'un réservoir (4) vers ledit canal d'expulsion (12) lors de chaque actionnement du distributeur, ledit dispositif de transfert (16, 17) exerçant une pression sur une partie du réservoir (4) lors de son actionnement, chaque réservoir comporte une coquille (4) comportant une surface supérieure (5), ladite coquille comportant une ouverture, opposée à ladite surface supérieure (5), et fermée hermétiquement par une paroi de fermeture (6) déchirable tel qu'une feuille d'aluminium, ladite paroi (6) étant adaptée à se déchirer sous l'effet d'une pression minimale exercée sur elle pour s'ouvrir totalement et de manière prédéterminée vers l'extérieur de la coquille, caractérisé en ce que ledit dispositif de transfert (16, 17) exerçant, lors de son actionnement, cette pression minimale sur ladite surface supérieure (5) de la coquille (4), ladite pression étant transmise à ladite paroi de fermeture (6) par un élément de transfert (1), sensiblement rigide et creux reliant lesdites surfaces, de sorte qu'aucune pression ne s'exerce sur le produit contenu dans ledit réservoir (4).

2. Dispositif selon la revendication 1, dans lequel ledit dispositif de transfert (16,17) comporte un poussoir (16) pourvu d'un doigt (17) adapté à s'appuyer sur une surface (5) d'un réservoir (4) à chaque actionnement, une poussée sur le poussoir (16) amenant ledit doigt (17) à exercer une pression sur ledit réservoir (4), ladite pression étant transmise à ladite paroi de fermeture (6) par ledit élément de transfert (1), ledit élément de transfert (1) assurant ainsi l'ouverture de ladite paroi de fermeture (6) et le transfert de la totalité de la dose de poudre vers le canal d'expulsion (12) du distributeur de produit.

3. Dispositif selon la revendication 1 ou la revendication 2, dans lequel ledit élément de transfert (1) est réalisé sous la forme d'au moins un insert rigide (1) disposé à l'intérieur de chaque réservoir (4).

4. Dispositif selon la revendication 3, dans lequel ledit insert (1) est creux et a une structure ouverte, la forme et les dimensions extérieures de l'insert étant telles que l'insert (1) est en contact avec le milieu de la paroi de fermeture (6), sur toute la longueur de celle-ci, et que la section maximale de l'insert (1) correspond environ à la surface de ladite paroi de fermeture (6).

5. Dispositif selon la revendication 3 ou la revendication 4, dans lequel ledit insert (1) comporte des anneaux parallèles (la) reliés par des segments longitudinaux (1b).

6. Dispositif selon la revendication 3 ou la revendication 4, dans lequel l'insert (1) est un ressort hélicoïdal.

7. Dispositif selon la revendication 3, dans lequel l'insert (1) est formé d'un tube rigide et creux qui est adapté à contenir la dose de poudre destinée à chacun des réservoirs (4).

8. Dispositif selon l'une quelconque des revendications précédentes, dans lequel les réservoirs (4) sont disposés radialement sur un disque rigide (50).

9. Dispositif selon l'une quelconque des revendications 1 à 7, dans lequel les réservoirs (4) sont disposés parallèlement les uns aux autres sur une bande (51).

## Patentansprüche

1. Vordosier-Einrichtung (2) für ein pulverförmiges Produkt, das in einer Abgabevorrichtung für das Produkt enthalten ist, die einen Ausstoßkanal (12) umfaßt, wobei die Vorrichtung wenigstens einen Behälter (4) aufweist, von denen jeder eine Dosis des Produktes einschließt, die dazu bestimmt ist, bei jeder Betätigung der Abgabevorrichtung ausgestoßen zu werden, wobei die Abgabevorrichtung eine Überführungseinrichtung (16, 17) aufweist, die dazu dient, bei jeder Betätigung der Abgabevorrichtung die Gesamtheit einer Dosis des Produktes aus einem Behälter (4) zum Ausstoßkanal (12) zu überführen, wobei die Überführungseinrichtung (16, 17) einen Druck auf einen Teil des Behälters (4) bei ihrer Betätigung ausübt, wobei jeder Behälter eine Schale (4) umfaßt, die eine obere Oberfläche (5) aufweist, und die Schale eine Öffnung besitzt, die der oberen Oberfläche (5) gegenüber liegt und hermetisch durch eine zerreißbare Verschlußwand (6), wie z.B. eine Aluminiumfolie verschlossen ist, wobei die Wand (6) unter der Wirkung eines sehr kleinen, auf sie ausgeübten Drucks zerreißen kann, um sich vollständig und in vorbestimmter Weise zur Außenseite der Schale hin zu öffnen, dadurch **gekennzeichnet,** daß die Überführungseinrichtung (16, 17) bei ihrer Betätigung diesen sehr geringen Druck auf die obere Oberfläche (5) der Schale (4) ausübt, wobei dieser Druck auf die besagte Verschlußwand (6) durch ein Übertragungselement (1) übertragen wird, das im wesentlichen starr und hohl ist und diese Oberflächen derart miteinander verbindet, daß keinerlei Druck auf das in dem Behälter (4) enthaltene Produkt ausgeübt wird.

2. Vorrichtung nach Anspruch 1, bei der die Übertragungseinrichtung (16, 17) einen Drücker (16) umfaßt, der mit einem Finger (17) versehen ist, der geeignet ist, sich bei jeder Betätigung an einer Oberfläche (5) eines Behälters (4) abzustützen, wobei ein Druck auf den Drücker (16) den Finger (17) dazu bringt, einen Druck auf den Behälter (4) auszuüben, wobei dieser Druck auf die Verschlußwand (6) durch das Übertragungselement (1) übertragen wird, wobei das Übertragungselement (1) auf diese Weise das Öffnen der Verschlußwand (6) und die Überführung der Gesamtheit der Pulverdosis zum Ausstoßkanal (12) der Abgabevorrichtung für das Produkt sicherstellt.

3. Vorrichtung nach Anspruch 1 oder 2, bei der das Übertragungselement (1) in Form von wenigstens einem starren Einsatz (1) ausgeführt ist, der im Inneren eines jeden Behälters (4) angeordnet ist.

4. Vorrichtung nach Anspruch 3, bei der der Einsatz (1) hohl ist und eine offene Struktur besitzt, wobei die Form und die Außenabmessungen des Einsatzes so beschaffen sind, daß der Einsatz (1) mit der Mitte der Verschlußwand (6) über deren gesamte Länge in Berührung steht, und daß der maximale Querschnitt des Einsatzes (1) ungefähr der Oberfläche der Verschlußwand (6) entspricht.

5. Vorrichtung nach Anspruch 3 oder 4, bei der der Einsatz (1) parallele Ringe (1a) umfaßt, die durch Längssegmente (1b) verbunden sind.

6. Vorrichtung nach Anspruch 3 oder 4, bei der der Einsatz eine Schraubenfeder ist.

7. Vorrichtung nach Anspruch 3, bei der der Einsatz (1) von einem starren und hohlen Rohr gebildet wird, das geeignet ist, die Dosis des Pulvers zu enthalten, die für jeden der Behälter (4) bestimmt ist.

8. Vorrichtung nach einem der vorhergehenden Ansprüche, bei der die Behälter (4) radial auf einer starren Scheibe (50) angeordnet sind.

9. Vorrichtung nach einem der Ansprüche 1 bis 7, bei der die Behälter (4) parallel zu einander auf einem Streifen (51) angeordnet sind.

## Claims

1. A device (2) for pre-dosing a powder disposed in a dispenser that includes an expulsion channel (12), the device comprising at least one reservoir (4) each containing a dose of powder that is to be expelled on each actuation of the dispenser, said dispenser including a transfer device (16, 17) designed to transfer an entire dose of powder from reservoir (4) towards said expulsion channel (12) on each actuation of the dispenser, said transfer device (16, 17), when actuated, exerting pressure on a portion of a reservoir (4), each reservoir including a shell (4) having a top surface (5), said shell including an opening opposite said top surface (5) that is hermetically closed by a tearable closure wall (6) such as an aluminum foil, said wall (6) being adapted to tear under the effect of a minimum pressure being exerted thereon to open totally and in a predetermined manner towards the outside of the shell, characterized in that said transfer device (16, 17), when actuated, exerting said minimum pressure on said top surface (5) of the shell (4), said pressure being transmitted to said closure wall (6) by a transfer element (1), which is substantially rigid and hollow and which interconnects said surfaces so that no pressure is exerted on the powder contained in said reservoir (4).

2. A device according to claim 1, in which said transfer device (16, 17) comprises a pushbutton (16) provided with a finger (17) adapted to press on one surface (5) of a reservoir (4) on each actuation, thrust on the pushbutton (16) causing said finger (17) to exert pressure on said reservoir (4), said pressure being transmitted to said closure wall (6) by said transfer element (1), said transfer element (1) thus causing said closure wall (6) to open and transferring all of the dose of powder towards the expulsion channel (12) of the dispenser.

3. A device according to claim 1 or claim 2, in which said transfer element (1) is made in the form of at least one rigid insert (1) disposed inside each reservoir (4).

4. A device according to claim 3, in which said insert (1) is hollow and open in structure, the outside dimensions and shape of the insert being such that the insert (1) is in contact with the middle of the closure wall (6) along the entire length thereof, and such that the maximum section of the insert (1) corresponds approximately to the surface of said closure wall (6).

5. A device according to claim 3 or claim 4, in which said insert (1) comprises parallel rings (la) interconnected by longitudinal segments (1b).

6. A device according to claim 3 or claim 4, in which the insert (1) is a helical spring.

7. A device according to claim 5, in which the insert (1) is in the form of a hollow rigid tube adapted to contain the dose of powder intended for each of the reservoirs (4).

8. A device according to any preceding claim, in which the reservoirs (4) are disposed radially on a rigid disk (50).

9. A device according to any one of claims 1 to 7, in which the reservoirs (4) are disposed parallel to one another on a strip (51).
